# EUROPEAN PATENT SPECIFICATION

(11) **EP 1 481 692 B1**
(45) Date of publication and mention of the grant of the patent: **28.03.2007**
(21) Application number: 02765424.3
(22) Date of filing: 05.09.2002
(51) Int. Cl.: A61L 2/06, A61B 1/00, A61L 2/26, A61L 2/20

(54) **STERILIZER**
STERILISATOR
STERILISATEUR

(30) Priority: 11.03.2002 JP 2002065883
(43) Date of publication of application: 01.12.2004
(73) Proprietor: Olympus Corporation, Tokyo 151-0072 (JP)
(72) Inventor: HASEGAWA, Hitoshi, Kohoku-ku, Yokohama-shi, Kanagawa 222-00 (JP); SUZUKI, Eiri, Sagamihara-shi, Kanagawa 229-0038 (JP); KUROSHIMA, Hisashi, 243 Olympus-Nishihachioji-Corp, Hachioji-shi, Tokyo 193-0832 (JP); NOGUCHI, Toshiaki, Tachikawa-shi, Tokyo 190-0002 (JP)
(74) Representative: von Hellfeld, Axel
(86) International application number: PCT/JP2002/009060
(87) International publication number: WO 2003/075963

(56) References cited:
- WO-A-00/59552
- DE-A1- 19 724 133
- JP-A- 5 337 081
- JP-A- 5 337 082
- JP-A- 5 337 171
- JP-A- 2000 051 323
- JP-A- 2002 017 824
- JP-A- 2002 046 772
- JP-A- 2002 253 477
- JP-A- 2002 253 648
- JP-A- 2002 325 719
- JP-B2- 60 036 297
- US-A- 5 609 561
- US-A- 5 906 801
- US-A1- 2002 001 551

## Description

### Technical Field

The present invention relates to a sterilization apparatus.

### Background Art

The first prior art will be described first. In general, before an object to be sterilized is placed in the chamber of a high pressure steam sterilization apparatus (to be referred to as an autoclave) or a gas sterilization apparatus such as an EOG sterilization apparatus, the object is stored in a case, called a cast, made of metal or the like, maintain the shape of the apparatus or stored in a gas-permeable resin pack, called a peel pack, to maintain a sterilized state after sterilization.

The second prior art will be described next. In general, endoscopic examination is performed for five to 20 cases per facility. In order to efficiently perform endoscopic examination, a cleaning/disinfection process or cleaning/sterilization process must be efficiently performed for the endoscope within the time intervals between the cases. In the present circumstances, preliminary cleaning based on a manual and main cleaning/sterilization using an automatic endoscope cleaning/disinfecting device are performed. This operation is performed in the following way.

When endoscopic examination is complete, preliminary cleaning (cleaning the outer surface of the endoscope and supplying air/water into the channel) is performed on the bed side, and the endoscope is set in the automatic endoscope cleaning/disinfecting device. The automatic endoscope cleaning/disinfecting device has a function for cleaning and rinsing the outer surface of the scope and the interior of the channel, and a function for disinfecting the scope with an antiseptic solution afterward and performing side rinsing. In addition, there is available a product having a function for drying the interior of the channel.

Recently, with the emergence of new pathogens and the like, the importance of endoscope sterilization has risen. In the present circumstances, therefore, a sterilization process is performed by using an EOG gas or the like. In consideration of the effects of EOG gas on the human body and its effects on the environment, an autoclave apparatus has been increasingly used, which poses less problems in terms of environment.

An endoscope incorporates many precision components, and hence has been considered to be difficult to sterilize in the above autoclave. Recently, however, as the heat resistance of components and adhesives has improved, an endoscope which causes no problems in terms of resistance even under the conditions of, for example, 135°C and 5 min, which are autoclave conditions, has begun to be developed.

The third prior art will be described next. In an autoclaving process, in order to maintain a sterilized state after an object is sterilized, the object is set in a sterilization apparatus after being packed in a packing member, and is then subjected to a sterilization process.

Problems in the first prior art will be described. Conventionally, storing work requires another workbench. This is an undesirable state for a narrow space for medical practice. In addition, such a workbench is contaminated because an object to be sterilized before a sterilization process is placed on it. It is therefore necessary to independently disinfect or sterilize the workbench, resulting in more work.

A problem in the second prior art will be described next. When an endoscope is to be mounted in the general autoclave apparatus described in the prior art, the following process is required: clean the endoscope, mount the endoscope on a sterilization tray dedicated for endoscopes in another place, and load the endoscope in the autoclave apparatus installed in another place. This process takes much labor, and hence a very long time is required for a cleaning/ sterilization process between cases. This hinders efficient execution of examination. That is, there is a considerable time interval between the instant at which one case is complete and the instant at which the next case is started, resulting in a great decrease in the number of examinations that can be conducted per day. This also leads to economic inefficiency.

A problem in the third prior art will be described next.

Conventionally, packing work for an object to be sterilized is performed away from where a sterilization apparatus is installed, and the packed object is carried and attached to the sterilization apparatus. This hinders improvements in the efficiency of a sterilization process.

Other prior art arrangements are known from documents DE 197 24 133 A1, US 5 906 801 A, WO 00/59552 A, US 2002/001551 A1 and US 5 609 561 A. In all of these cases, however, the above problems of associated packing work still exist.

### Disclosure of Invention

It is a first object of the present invention to provide a sterilization apparatus which allows the effective use of a work space for medical practice, facilitates work before sterilization, and can enhance sanitary supervision.

It is a second object of the present invention to provide a sterilization apparatus which can shorten the time for preparation for the storage of an endoscope in an autoclave apparatus, improves the ease of use for a user, and can execute efficient endoscopic examination.

It is a third object of the present invention to provide a sterilization apparatus which can improve the working efficiency of a sterilization process by performing packing work for an object to be sterilized near a sterilization apparatus.

According to a first aspect of the present invention, there is provided a sterilization apparatus having the features recited in claim 1. The sterilization apparatus of the invention comprises:
a chamber in which an object to be sterilized is stored and a sterilization process is performed; and
a work space forming unit which forms a space for execution of a process of storing the object in the chamber upon setting the object in a predetermined storage state and can be stored in the chamber, wherein the work space forming unit comprises:
   a workbench which is stored/locked in the chamber so as to be freely pulled out therefrom, is set at a predetermined position in a pulled-out state, and allows the object to be placed and execution of a process of storing the object in the chamber, and
   a setting unit which sets the object in a predetermined state for execution of a sterilization process.

According to the second aspect of the present invention, there is provided a sterilization apparatus according to claim 1, further comprising an operation member which reduces a working load of returning the workbench from a pulled-out position to a storing position.

According to the third aspect of the present invention, there is provided a sterilization apparatus according to claim 2, wherein the operation member comprises a foot-operated pedal.

According to the fourth aspect of the present invention, there is provided a sterilization apparatus according to claim 1, wherein the setting unit includes a rotating surface substantially parallel to a base surface of the workbench.

According to the fifth aspect of the present invention, there is provided a sterilization apparatus according to claim 1, wherein the work space forming unit includes a sterilization tray which can be moved into and out of the chamber by a slide member, and the sterilization tray includes a sterilization tray lid which is used as a workbench including a setting unit for setting the object in a predetermined state for execution of a sterilization process.

According to the sixth aspect of the present invention, there is provided a sterilization apparatus according to claim 1, wherein the setting unit further comprises:
packing means for packing the object, and
sealing means for sealing the packing means.

According to the seventh aspect of the present invention, there is provided a sterilization apparatus according to claim 1, wherein the packing means includes a seat portion on which the object and a packing member which packs the object are placed, and the sealing means comprises a lid portion which seals and locks the packing member packing the object, and a hinge portion which allows the lid portion to be opened/closed.

According to the eighth aspect of the present invention, there is provided a sterilization apparatus according to claim 7, wherein the lid portion further includes a welding heat transfer portion for sealing the packing member, and a hook portion for fixing the welding heat transfer portion on the seal portion when the lid portion is closed.

According to the ninth aspect of the present invention, there is provided a sterilization apparatus according to claim 1, which includes a door for opening/closing the chamber, and in which a welding heat transfer portion for sealing a packing member which packs the object is placed inside the door, a packing is mounted on a portion in the chamber to which the welding heat transfer portion is fitted, and the packing member is welded/sealed by bringing the packing into tight contact with the welding heat transfer portion with the packing member being clamped therebetween when the door is closed.

According to the tenth aspect of the present invention, there is provided a sterilization apparatus according to claim 1, which includes a door for opening/closing the chamber, and in which a welding device for sealing a packing member which packs the object is placed inside the door, the welding device has a hinge shape including an upper portion which opens/closes in a vertical direction and a lower portion fixed to the door, a heat transfer portion is provided for the upper portion, a packing is formed on the lower portion which is fitted to the upper portion when the upper portion closes, and the packing member is welded/sealed by clamping the packing member between the heat transfer portion and the packing.

### Brief Description of Drawings

FIGS. 1A to 1C are views showing the arrangement of a sterilization apparatus 50 according to the first embodiment of the present invention;
FIG. 2 is a block diagram showing the internal arrangement of the sterilization apparatus 50;
FIG. 3 is a flowchart for explaining a sequence for a high pressure steam sterilization (autoclaving) process by the sterilization apparatus 50;
FIG. 4 is a timing chart showing how the pressure in a chamber 2 changes with the progression of a sterilization process and the timings at which the respective constituent elements described above are turned on and off or opened or closed;
FIG. 5 is a schematic view showing an embodiment of a link mechanism which links a workbench 3 to a foot-operated pedal 5;
FIG. 6 is a view showing an embodiment in which hooks 12 are provided to directly fix an object 4 to be sterilized to the workbench 3 stored in the chamber 2;
FIGS. 7A and 7B are views showing an embodiment in which a rotating table 13 is incorporated in the workbench 3;
FIGS. 8A and 8B are views showing the arrangement of a sterilization apparatus according to the second embodiment of the present invention;
FIG. 9 is a view showing a chamber portion extracted from an autoclave apparatus described with reference to FIG. 8A and 8B and a sterilization tray 112;
FIG. 10 is a view showing an outer appearance of a sterilization apparatus 201 according to the third embodiment of the present invention;
FIG. 11 is an enlarged view of a packing workbench 205 of the sterilization apparatus 201 according to the third embodiment;
FIG. 12 is a view showing a state wherein an object 215 to be sterilized and a packing member 216 are placed on a packing workbench 205;
FIG. 13 is a view showing a state wherein the object 215 and packing member 216 are placed on the packing workbench 205, and a lid portion 207 is closed;
FIG. 14 is a view showing the arrangement of a sterilization apparatus according to the fourth embodiment of the present invention;
FIG. 15 is a view showing a packing member in the fourth embodiment;
FIG. 16 is a view showing the arrangement of a sterilization apparatus according to the fifth embodiment of the present invention;
FIG. 17 is a view showing a packing member in the fifth embodiment;
FIG. 18 is an enlarged view of a welding device 235;
FIG. 19 is a view showing the arrangement of a sterilization apparatus 301 according to the sixth embodiment of the present invention; and
FIG. 20 is a view showing a modification of the arrangement of the sterilization apparatus 301 shown in FIG. 19.

### Best Mode for Carrying Out the Invention

### (First Embodiment)

In the first embodiment, a workbench is stored in the chamber of a sterilization apparatus, and is taken out from the chamber to be used as a workbench when a pre-sterilization process is to be performed for an object to be sterilized. The object set on the workbench or peel-packed is stored together with the workbench in the chamber, and hence is sterilized together with the workbench, which saves space as well.

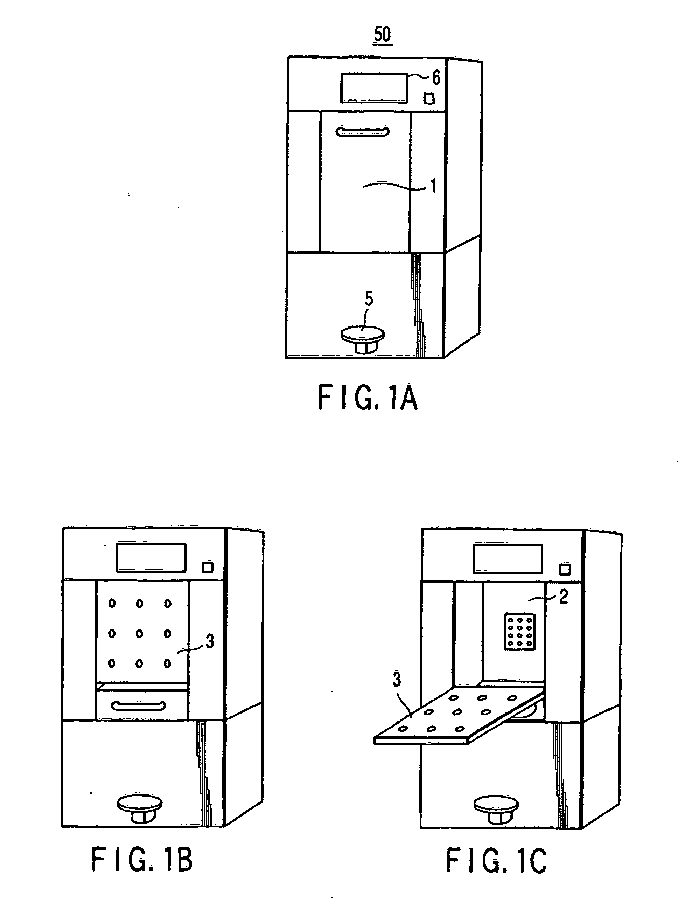

The first embodiment of the present invention will be described in detail below with reference to the views of the accompanying drawing. FIGS. 1A. 1B, and 1C are views showing the arrangement of a sterilization apparatus according to the first embodiment. FIG. 1A shows a state wherein a door 1 of a sterilization apparatus 50 is closed. FIG. 1B shows a state wherein the door 1 is moved downward to be open. FIG. 1C shows a state wherein a workbench 3 is taken out from a chamber 2. Referring to FIG. 1A, reference numeral 5 denotes a foot-operated pedal; and 6, an operation panel.

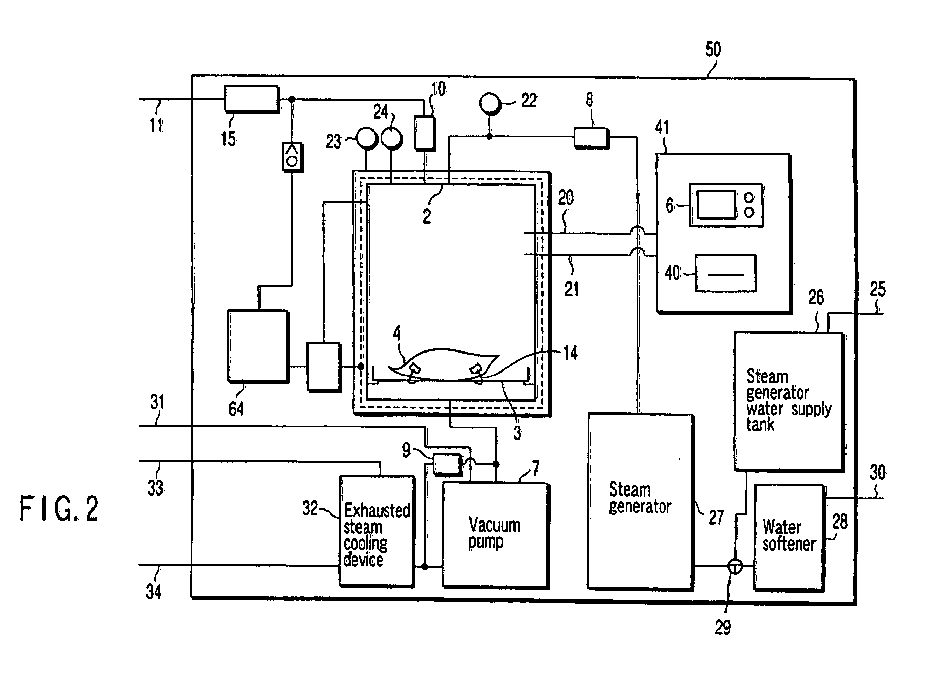

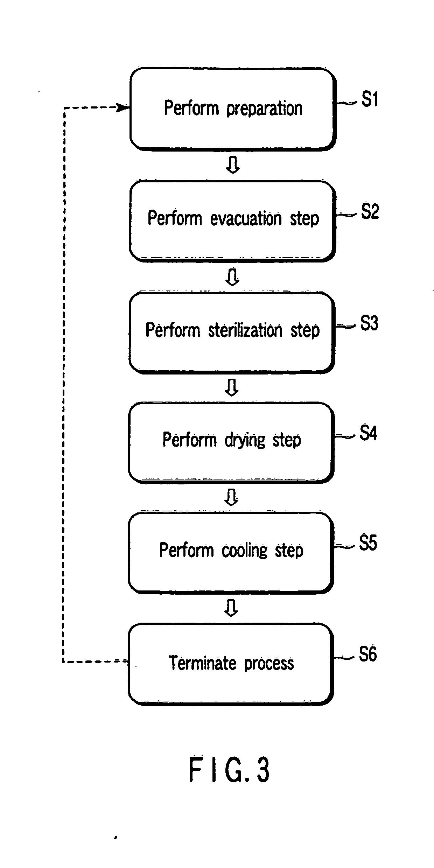

FIG. 2 is a block diagram showing the internal arrangement of the sterilization apparatus 50. FIG. 3 is a flowchart for explaining a sequence for an autoclaving process by the sterilization apparatus 50. An autoclaving process in this embodiment will be described with reference to FIGS. 1A to 3.

First of all, in a preparation step (step S1), an operator opens the door 1 by pressing a switch on the operation panel 6. The operator tilts the workbench 3 to an almost horizontal position. The operator performs the operation of storing an object 4 to be sterilized in a storing medium such as a cast or peel pack on the workbench 3. When operation such as packing is complete, the object is fixed on the workbench 3 by using a fixture 14 mounted on the cast or peel pack, and is stored together with the workbench 3 in the chamber 2 of the sterilization apparatus 50. If the workbench 3 is too heavy to be manually pushed up, the workbench 3 may be coupled to the foot-operated pedal 5 through a link mechanism to allow the workbench 3 to be stored with light pedaling operation.

When the workbench 3 is stored, the operator closes the door 1 by operating a switch on the operation panel 6 again. Upon confirming that the door 1 is closed, the operator presses a step start switch on the operation panel 6. With this operation, the door 1 of the sterilization apparatus 50 is locked, and a sterilization process is started. In the sterilization process, first of all, the pressure in the chamber 2 is reduced by an evacuation step (step S2) of evacuating the chamber 2 with a vacuum pump 7. When the pressure in the chamber 2 is reduced to a preset pressure (e.g., -0.09 MPa), the vacuum pump 7 is stopped. A steam supply valve 8 is then opened to introduce high-pressure steam generated by a steam generator 27 into the chamber 2, and a sterilization process (step S3) is started. Steam is supplied into the chamber 2 until the temperature therein reaches a sterilization temperature (e.g., 135°C). In order to improve efficiency, evacuation and steam supply may be alternately repeated.

When the temperature in the chamber 2 reaches the set sterilization temperature, the temperature in the chamber 2 is maintained for a preset sterilization time (e.g., 5 min). When the sterilization time has elapsed, a steam exhaust valve 9 is opened to exhaust high-pressure steam in the chamber 2 outside the chamber 2. The exhausted high-pressure steam cooled to water by cooling water supplied from a cooling water inlet 33 in an exhausted steam cooling device 32, and is drained from a drain outlet 34.

After the steam is exhausted, the steam exhaust valve 9 is closed, and the chamber 2 is evacuated again by the vacuum pump 7. This is a drying step (step S4), in which since the chamber 2 is evacuated at a high temperature, water droplets are quickly vaporized and dried.

When a preset drying time (e.g., 10 min) has elapsed, the vacuum pump 7 is stopped, and an intake valve 10 is opened to return the pressure in the chamber 2 to atmospheric pressure. In order to prevent the interior of the chamber 2 from being contaminated by normal bacteria floras in the air, a filter having a filter mesh of 0.2 µm or less, which is generally called a sterile filter 15, is attached to an outside air inlet 11 of the intake valve 10. When the pressure in the chamber 2 returns to atmospheric pressure, the sterilization process is complete. The operator then opens the door 1 by operating a switch on the operation panel 6 and can take out the object 4 from the chamber 2.

Note that since the object 4 may be kept at a high temperature immediately after the step, the object 4 is cooled in the chamber 2 or in another place (step S5).

Referring to FIG. 2, reference numeral 64 denotes a compressor; 21, 22, 23, and 24, pressure gages; and 20, a temperature sensor. The operation panel 6 and a recording device 40 constitute a control box 41. A steam generator water supply tank 26 coupled to a manual water inlet 25 and a water softener 28 coupled to a steam water inlet 30 are connected to the steam generator 27 via a switching valve 29.

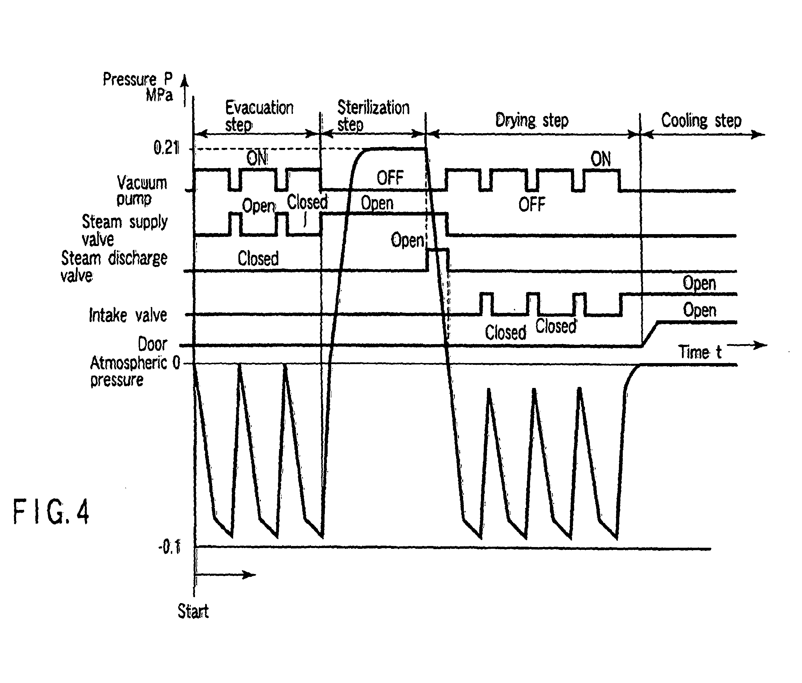

The timing chart of FIG. 4 shows how the pressure in the chamber 2 changes with the progress of the above sterilization process and the timings at which the respective constituent elements described above are turned on and off or opened and closed. Note that in the ON period of the vacuum pump 7, the vacuum pump 7 is repeatedly turned on and off. The ON and OFF timings correspond to the atmospheric pressure.

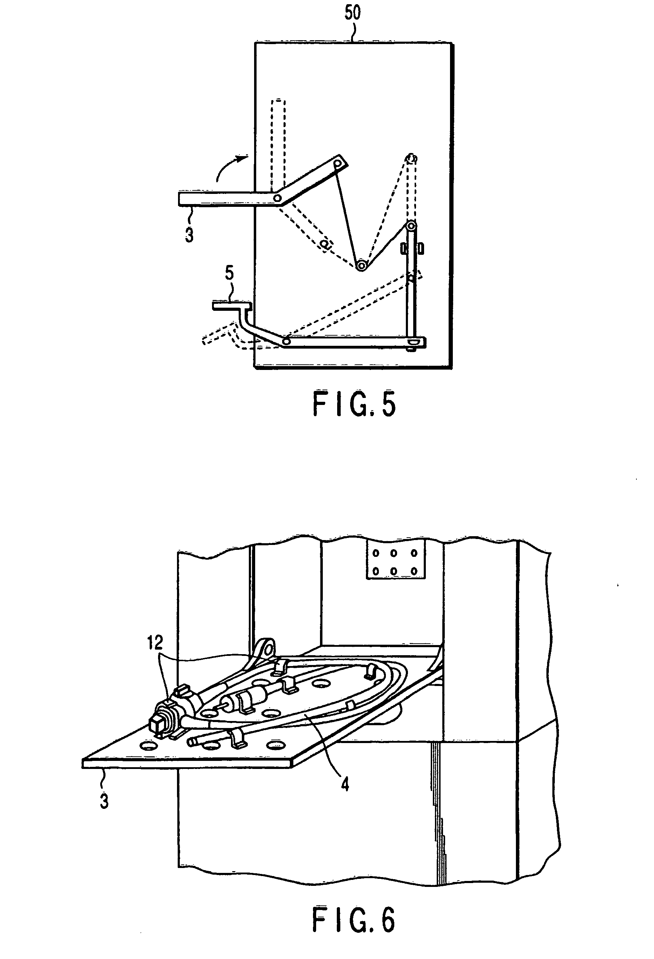

FIG. 5 is a schematic view showing an embodiment of a link mechanism which links the above workbench 3 to the foot-operated pedal 5.

FIG. 6 shows an embodiment in which the workbench 3 stored in the chamber 2 has hooks 12 for directly fixing the object 4 on the workbench 3. This arrangement is made in consideration of the ease of mounting the object 4. This arrangement is most suitable for mounting of a tool which has a complicated shape and is to be used immediately after sterilization. The arrangement requires no separate work space and allows the object 4 to be quickly mounted on the sterilization apparatus 50, thereby greatly improving the working efficiency.

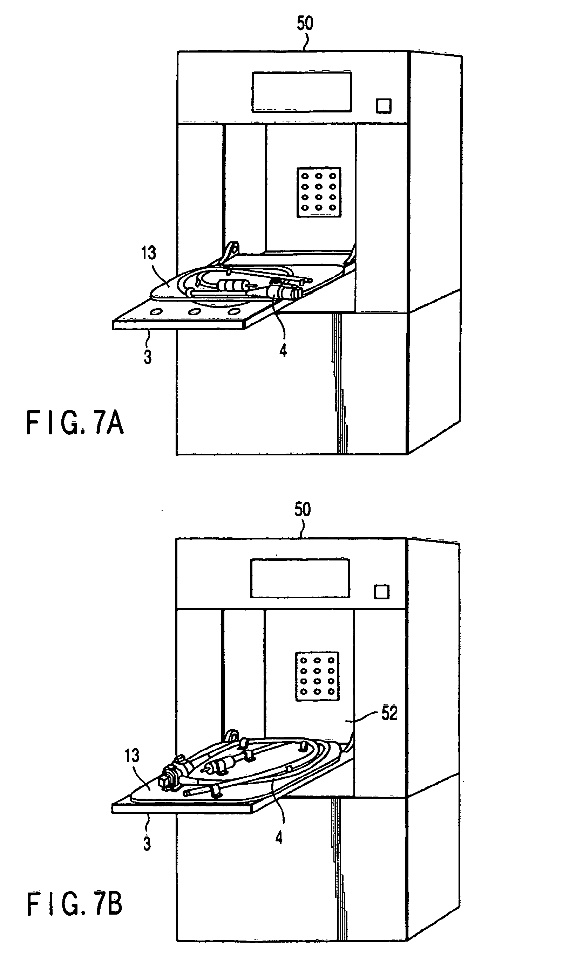

FIGS. 7A and 7B show an embodiment in which a rotating table 13 is incorporated in the workbench 3. Referring to FIG. 7A, when a long object 4 to be sterilized is to be mounted, the workbench 3 is rotated to the landscape position to facilitate mounting the object 4. Referring to FIG. 7B, when the object 4 is to be set in the sterilization apparatus 50 after being mounted on the workbench 3, the rotating table 13 is rotated in the longitudinal direction in accordance with a vertically oriented chamber 52. Although the vertically oriented chamber 52 can be smaller in occupied floor area than a horizontally oriented chamber, the workbench 3 becomes vertically oriented as well, which makes it difficult for the operator to reach the back of the workbench. Providing the rotating table 13, however, facilitates mounting the object 4 in the vertically oriented chamber 52.

Note that the workbench 3, hooks 12, and the like constitute a work space forming unit. In addition, the rotating table 13 forms a setting unit for setting the object 4 in a predetermined state.

According to the first embodiment, the object 4 can be mounted in front of the sterilization apparatus 50 and can be directly hooked on the sterilization apparatus 50, and hence can be easily loaded and unloaded. In addition, since the workbench 3 is stored in the apparatus during a sterilizing operation, space saving can be realized. Furthermore, since the workbench 3 is also sterilized, there is no need to separately disinfect or sterilize the workbench 3. This can contribute to an improvement in working efficiency and space saving.

The chamber 2 of the sterilization apparatus 50 is preferably designed to be vertically oriented so as to reduce the occupied floor area of the apparatus. When a long object 4 to be sterilized, which has a complicated shape, is to be mounted in the chamber 2, the operator can mount the object on a front portion of the workbench 3 without reaching its back by rotating the workbench 3 to the landscape position. This can greatly improve the working efficiency.

### (Second Embodiment)

In the second embodiment, a sterilization tray (conventional tray) for storing an object to be sterilized such as an endoscope and holding it in a sterilized state after sterilization is provided with a working means which can facilitate packing operation and setup of an object to be sterilized (in a specific embodiment, an endoscope holding member is provided on a sterilization tray lid).

After an object to be sterilized such as an endoscope is cleaned, the object is directly mounted on the sterilization tray near the autoclave apparatus and is automatically set up in the chamber. With this series of operations, the object can be easily mounted in the autoclave apparatus, and the total time required for the conventional sterilization process can be shortened, thereby efficiently executing endoscopic examination.

The second embodiment of the present invention will be described in detail below with reference to the views of the accompanying drawing.

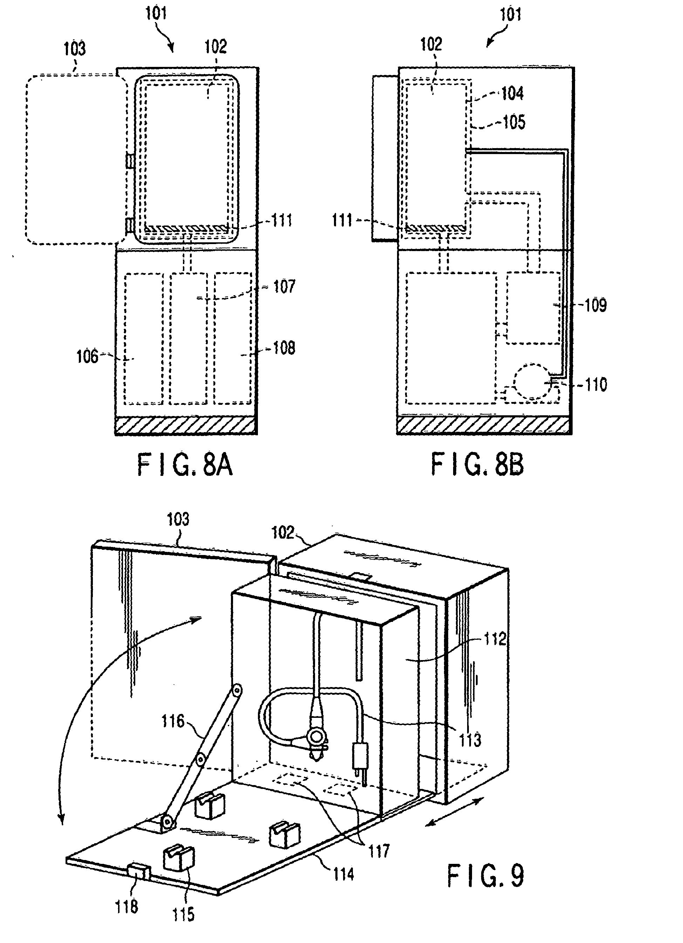

FIGS. 8A and 8B are views showing the arrangement of the autoclave apparatus according to the second embodiment. An autoclave apparatus body 101 includes a high pressure vessel (to be referred to as a chamber body 102 hereinafter) in which an object to be sterilized is to be inserted (set) and a chamber lid 103 which keeps the chamber body 102 in an airtight state and is opened/closed to load/unload the object. In the chamber body 102, a steam generator 109 for generating high-pressure steam at the time of execution of autoclaving is connected to a steam water supply tank 106 and is further connected to an inner chamber can 104 and outer chamber can 105 of the chamber body 102 via a steam supply piping.

A vacuum pump 110 is connected to the inner chamber can 104 via an exhaust piping. The vacuum pump 110 is used to perform a pre-process with high thermal efficiency before the execution of autoclaving by evacuating the chamber once and then replacing the air with high-pressure steam. The vacuum pump 110 is also used to promote drying of the object after autoclaving.

The vacuum pump 110 used for the autoclave apparatus generally exhausts steam, and hence a water ring pump is used. A vacuum pump tank 108 for supplying water required for the operation of the vacuum pump 110 is mounted in the autoclave apparatus.

When a sterilization step is complete in an autoclaving step, steam in the chamber is exhausted outside the chamber via a steam exhaust piping. Since this steam is at a very high temperature, the steam must be cooled to a certain extent. For this purpose, a steam cooling tank 107 for cooling steam is also mounted in the autoclave apparatus.

Although, in addition to the above components, this apparatus uses many components such as a control board for controlling the respective units, various kinds of electromagnetic valves, temperature sensors, and safety valves, a description thereof will be omitted.

The operation of the autoclave apparatus will be described next. First of all, an object to be sterilized is stored in the chamber body 102, and a chamber lid 103 is reliably closed. In general, a seal member (made of, for example, silicone rubber) driven by a compressor is provided at the contact portion between the chamber lid 103 and the chamber body 102 to reliably seal the chamber lid 103 and chamber body 102, thereby preventing steam from leaking at high pressure.

When a sterilization start switch (not shown) is turned on, the vacuum pump 110 operates to evacuate the inner chamber can 104. This operation is performed to prevent sterilization failure which is caused when there are portions (cold spots) of the interior of the chamber body 102 and object with which high=pressure steam does not reliably come into contact.

When a vacuum (e.g., about -0.1 MPa) is created in the chamber body 102, high-pressure steam is supplied into the inner chamber can 104 by the steam generator 109. When steam is properly supplied and the tempera= ture of the interior of the chamber body 102 and object reaches, for example, 135°C which is a sterilization condition, a sterilization timer (not shown) starts, and a sterilization step is performed at, for example, 135°C for 5 min.

When the 5-min sterilization step is complete, steam in the inner chamber can 104 is exhausted into the steam cooling tank 107 via the steam exhaust piping, and a vacuum pump 116 is operated again to dry the object. Steam is supplied into the outer chamber can 105 while the apparatus in the standby state. When these drying steps are complete and the object is naturally cooled, all the steps are complete.

FIG. 9 is a view showing a chamber portion extracted from the autoclave apparatus described with reference to FIG. 8A and 8B and a sterilization tray 112. This embodiment further includes a slide member 111 which houses the sterilization tray 112 of the scheme of installing an endoscope as an object to be sterilized in the longitudinal direction (hanging) in accordance with the shape of the endoscope, and moves the sterilization tray 112 outside the chamber. The slide member 111 is comprised of, for example, a slide rail and a driving motor.

As shown in FIG. 9, endoscope holding members 115 for holding an endoscope as an object to be sterilized are provided on a sterilization tray lid 114 of the sterilization tray 112. Although three endoscope holding members are provided in this embodiment, an arbitrary number of endoscope holding members 115 can be provided. The endoscope holding members 115 are provided at positions to restrict the disposition of the endoscope so as prevent the curling of the insertion portion of the endoscope at the time of execution of autoclaving.

The arrangement and operation of the sterilization tray 112 will be described next. The sterilization tray 112 includes a door fixing stay 116 to allow the sterilization tray lid 114 on the front side to open forward and be fixed in a horizontal position. The sterilization tray lid 114 includes the endoscope holding members 115 for holding, for example, an endoscope, and is configured to also serve as a workbench for preparing (setting) the endoscope in this state. Although not shown, the sterilization tray body also includes ventilating holes for intaking and exhausting steam, a seal member for reliably holding a sterilized state, and the like.

The sterilization tray 112 has a structure which allows the tray to be detachably mounted on the slide member 111 of the autoclave apparatus body 101 with magnets 117. The sterilization tray 112 can be moved into and out of the sterilization tray 112 before and after a sterilization step while being fixed on the slide member 111. The chamber is made of iron, and the magnets are fixed on the tray.

A series of sterilizing operations will be described next. The autoclave apparatus body 101 is in a standby state for the start of a sterilization step. The sterilization tray 112 is fixed to the slide member 111 of the autoclave apparatus body 101. The sterilization tray lid 114 is open. In this state, an endoscope 113 which has undergone a cleaning process is mounted on the endoscope holding members 115 by using the sterilization tray lid 114 in the open state as a workbench.

When the sterilization tray lid 114 to which the endoscope 113 is fixed is closed, the endoscope 113 is stored in the sterilization tray 112, and the lid is fixed with a clamp 118. FIG. 9 shows a state wherein the endoscope is set in the sterilization tray 112 and is mounted in the chamber. The sterilization tray 112 is then mounted in the chamber of the autoclave apparatus by using the moving mechanism of the slide member 111. The slide member 111 may be of a manual system or may be automatically operated in conjunction with an operation switch or opening/closing operation of the chamber lid of the autoclave apparatus.

The chamber lid 103 of the autoclave apparatus is then closed, and a sterilization step is started. A specific description of the sterilization step will be omitted.

When the sterilization step and drying step are complete, the chamber lid 103 is opened, and the sterilization tray 112 is moved out of the chamber of the autoclave apparatus by using the moving mechanism of the slide member 111. When the endoscope is to be used for diagnosis or the like at this point in time, the endoscope can be easily taken out from the chamber by opening the sterilization tray lid 114.

If the endoscope is not used soon after the above steps, since the slide member 111 and sterilization tray 112 are detachably mounted with the magnets 117 as described above, the endoscope can be removed from the slide member 111 together with the sterilization tray 112 and stored (without opening the sterilization tray lid). This makes it possible to hold the sterilized endoscope in the sterilized state.

According to the second embodiment,
1. An endoscope can be easily mounted in the chamber of the autoclave apparatus, and hence working efficiency improves.
2. Since the sterilization tray can also serve as a work space, space saving can be realized.

### (Third Embodiment)

The third embodiment is comprised of a storing unit which stores an object to be sterilized, a packing means for packing the storing unit, and a means for sealing the packing means.

Packing an object to be sterilized using the packing means and sealing the pack using the sealing means make it possible to save the labor of performing packing operation upon ensuring a packing work area in another place, transporting the packed object to a sterilization apparatus, and setting the object in the sterilization apparatus as in the prior art. This can realize efficient sterilizing operation.

The third embodiment of the present invention will be described in detail below with reference to the views of the accompanying drawing.

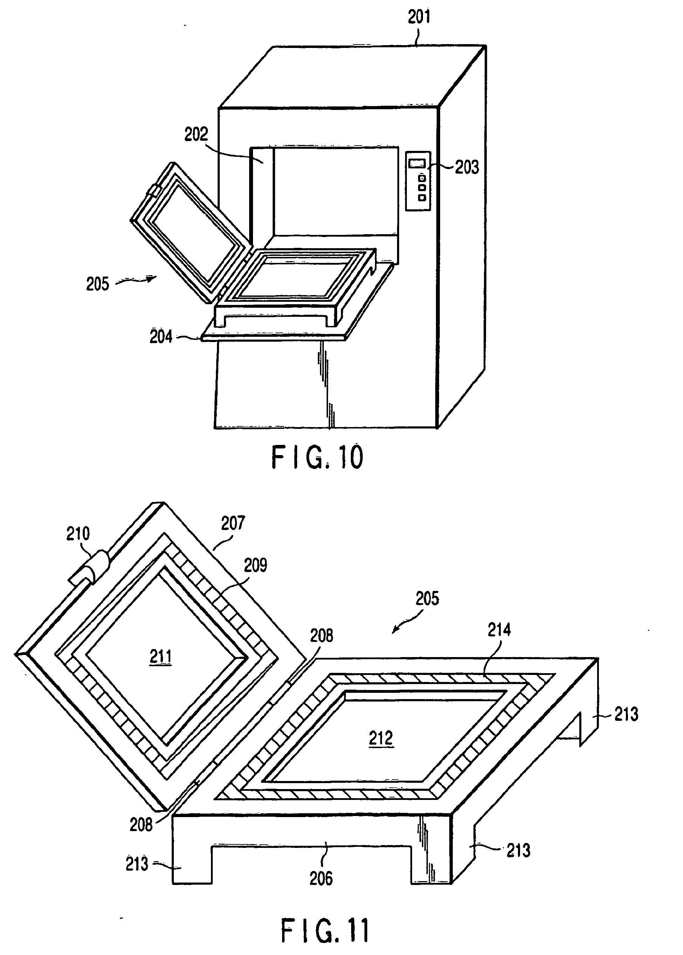

The third embodiment of the present invention will be described with reference to FIGS. 10 to 13. FIG. 10 shows an outer appearance of a sterilization apparatus 201 according to the third embodiment. An object to be sterilized is stored in a chamber 202 of the sterilization apparatus 201 to be subjected to a sterilization process. The sterilization apparatus 201 includes an operation panel 203, which is operated to operate the sterilization apparatus 201.

A door 204 is provided for the sterilization apparatus 201 to allow the chamber 202 to be opened/closed. The door 204 opens to a horizontal position. A packing workbench 205 used for packing an object to be sterilized is set/fixed on the inside of the door 204.

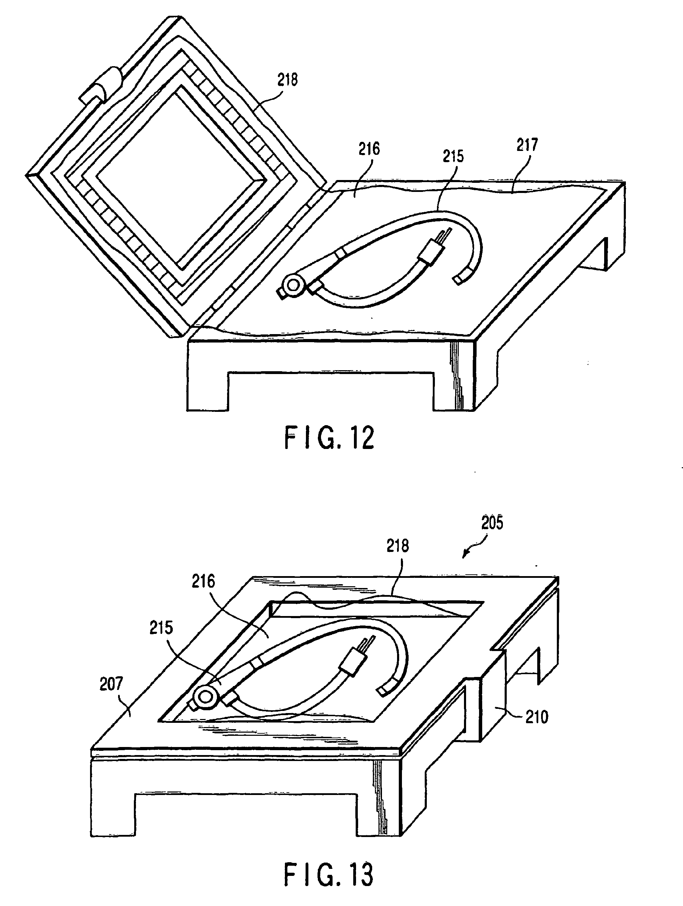

FIG. 11 is an enlarged view of the packing workbench 205 of the sterilization apparatus 201 according to this embodiment. The packing workbench 205 is comprised of a seat portion 206 on which an object to be sterilized and a packing member which packs the object are placed and a lid portion 207 which seals and locks the packing member packing the object. The lid portion 207 can be opened/closed through hinge portions 208.

The lid portion 207 includes a welding heat transfer portion 209 for sealing a packing member and a hook portion 210 for fixing the seat portion 206 when the lid portion 207 is closed. The welding heat transfer portion 209 is shaped to surround a central opening portion 211 of the lid portion 207, and generates heat, under the control of a control unit (not shown) of the sterilization apparatus 201, to weld a packing member which packs an object to be sterilized.

The central opening portion 211 of the lid portion 207 is shaped to allow steam supplied into the chamber 202 to be sufficiently applied to an object to be sterilized when the object is set on the packing workbench 205 so as to be subjected to a sterilization process in the chamber 202. A central portion 212 of the seat portion 206 is also open to allow steam to be sufficiently applied to the object in a sterilization process.

The seat portion 206 has four leg portions 213, i.e., a four-footed structure. An object to be sterilized is placed on the seat portion 206 so as to be lifted from the door 204 of the sterilization apparatus 201. This makes it possible to sufficiently apply steam to the object from below the seat portion 206. The seat portion 206 includes a packing 214 which is fitted to the welding heat transfer portion 209 of the lid portion 207 when the lid portion 207 is closed. This brings the welding heat transfer portion 209 into tight contact with the seat portion 206 to firmly clamp a packing member between them, thereby welding and sealing the packing member.

FIG. 12 is a view showing a state wherein an object 215 to be sterilized and a packing member 216 are placed on the packing workbench 205. The packing member 216 is comprised of a paper portion 217 and transparent film portion 218. One side of the paper portion 217 is bonded in advance to one side of the transparent film portion 218, but the three remaining sides of each portion are open. The paper portion 217 and transparent film portion 218 are equal in area, and are exactly overlaid on each other when they are folded along the bonded side as a center line.

FIG. 13 is a view showing a state wherein the object 215 and packing member 216 are placed on the packing workbench 205 and the lid portion 207 is closed. When the lid portion 207 is closed, the hook portion 210 is locked to fix the lid portion 207. This locks the object 215 to the packing workbench 205.

The effect of the above arrangement will be described next. In this embodiment, in order to pack the object 215 with the packing member 216 before a sterilization process, the packing member 216 is set on the packing workbench 205. As shown in FIG. 12, the paper portion 217 is spread on the seat portion 206, and the transparent film portion 218 is set on the lid portion 207 side in an open state, thereby setting the packing member 216 on the packing workbench 205 in an unfolded state.

At this time, the paper portion 217 is set on the seat portion 206 so as to cover the packing 214 which makes the central portion 212 fit to the welding heat transfer portion 209 of the lid portion 207 in order to allow the packing member 216 to properly pack and seal the object.

On the other hand, the transparent film portion 218 is also set on the lid portion 207 to cover the central opening portion 211 and welding heat transfer portion 209. Subsequently, the object 215 is set in a central portion of the paper portion 217 so as to be fitted in the central portion 212 of the seat portion 206. When the lid portion 207 is closed in this state, the object 215 is packed in the packing member 216, and the hook portion 210 is locked. As a consequence, the object 215 is locked to the packing workbench 205 while being packed in the packing member 216.

At this time, the object 215 is set in the central opening portion 211 of the lid portion 207 of the packing workbench 205 and the central portion 212, so that steam is sufficiently applied to the object when a sterilization process is performed. In addition, the welding heat transfer portion 209 of the lid portion 207 surrounds the object 215.

After the object 215 is placed on the packing workbench 205, the door 204 of the sterilization apparatus 201 is closed to set the object 215 in the chamber 202. The operation panel 203 is then operated to start a sterilization process. When the sterilization process is started, the welding heat transfer portion 209 of the packing workbench 205 is energized for a predetermined period of time to generate heat under the control of the control unit (not shown) of the sterilization apparatus so as to seal the packing member 216.

With this operation, the transparent film portion 218 in contact with the welding heat transfer portion 209 which surrounds the object 215 is welded to the paper portion 217. As a consequence, the packing member 216 is sealed, and the object 215 is packed in the packing member 216. Thereafter, high-temperature, high-pressure steam is supplied into the chamber 202 to sterilize the object 215.

At this time, since the 215 is placed in the central opening portion 211 of the lid portion 207 and the central portion 212 of the seat portion 206, steam is sufficiently applied to the object to reliably sterilize it. Note that the packing member 216 itself transmits steam, and hence steam passes through the packing member and comes into contact with the packed object 215 during a sterilization process, thereby sterilizing the object 215 by heating.

A user sets in advance a predetermined temperature and time for the sterilization apparatus 201 by operating the operation panel, and the apparatus performs a sterilization process on the basis of the set contents. When the sterilization process is terminated, a message indicating the end of the sterilization process is displayed on the operation panel. The user opens the door 204 of the sterilization apparatus 201 upon checking this display, and takes out the object 215 from the chamber 202. When the door 204 is opened, the object 215 is taken out from the chamber 202 while being locked to the packing workbench 205.

The lid portion 207 is then opened by unhooking the hook portion 210, and the object 215 is taken out. At this time, the object 215 is packed in the packing member 216, and the packing member 216 prevents adhesion of various bacteria to the object. This makes it possible to store the object in a sterilized state.

According to the third embodiment described above, the object 215 can be easily set in the sterilization apparatus 201 and can also be packed in a packing member, thereby reducing the working load.

### (Fourth Embodiment)

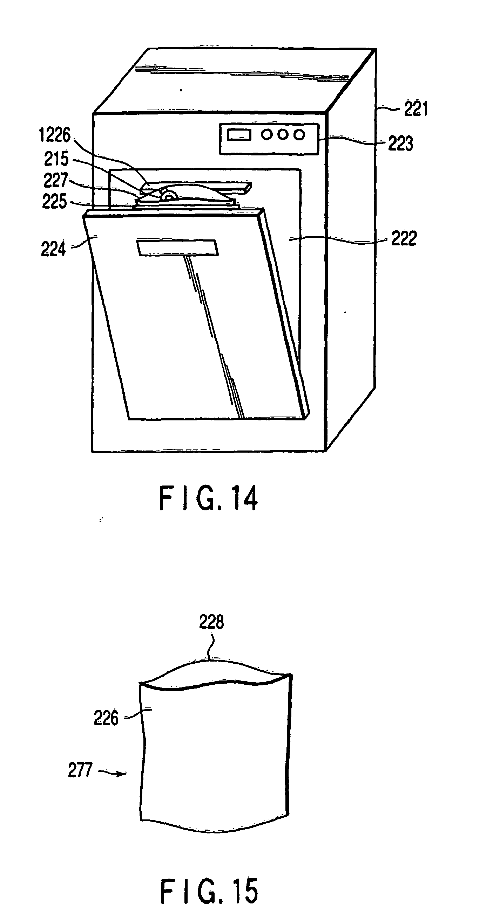

FIG. 14 is a view showing the arrangement of a sterilization apparatus according to the fourth embodiment. Referring to FIG. 14, a sterilization apparatus 221 includes a chamber 222 and operation panel 223. The sterilization apparatus 221 is operated by operating the operation panel 223. The sterilization apparatus 221 includes a door 224, which makes it possible to open/close the chamber 222. As shown in FIG. 14, the door 224 opens forward at a certain angle with respect to the lower side. When the door 224 is opened, a welding heat transfer portion 225 for sealing a packing member which packs an object 215 placed inside the apparatus appears.

A packing 1226 is mounted on a portion inside the chamber 222 to which the welding heat transfer portion 225 of the door 224 is fitted. This brings the door 224 into tight contact with the welding heat transfer portion 225 when the door 224 is closed, thereby firmly clamping the packing member between them and welding and sealing the packing member.

FIG. 15 is a view showing a packing member 226 in this embodiment. The packing member 226 in the embodiment is comprised of a paper portion 227 and transparent film portion 228. The two portions are overlaid and three sides of one portion are bonded to those of the other portion, thereby forming a bag.

In this embodiment, when a sterilization process is to be performed, the door 224 of the sterilization apparatus 221 is opened, and the packing member 226 is inserted between the door 224 and the chamber 222 with its opening portion facing up such that the transparent film portion 228 is located on the door 224 side and the paper portion 227 is located on the chamber 222 side, as shown in FIG. 14. The object 215 is then placed into the packing member 226 through the opening portion of the packing member 226. Thereafter, the door 224 is closed to bring the welding heat transfer portion 225 of the door 224 into tight contact with the packing 1226 in the chamber 222 while clamping the packing member 226.

The operation panel 223 is then operated to start a sterilization process. When a sterilization step is started, the welding heat transfer portion 225 of the door 224 is energized for a predetermined period of time under the control of the control unit (not shown) of the sterilization apparatus 221 to generate heat so as to seal the packing member 226. With this operation, the transparent film portion 228, with which the welding heat transfer portion 225 is in contact, is welded to the paper portion 227 of the packing member to seal the opening portion of the packing member 226, thereby packing the object 215 in the packing member 226.

Subsequently, high-temperature, high-pressure steam is supplied into the chamber 222 to sterilize the object 215. When a series of sterilizing operations is complete, a message indicating the end of the sterilization process is displayed on the operation panel 223. Upon checking this display, the user opens the door 224 of the sterilization apparatus 221 and takes out the object 215 from the chamber 222. At this time, the object 215 is packed in the packing member 226, and the packing member 226 prevents the adhesion of bacteria to the object 215. This makes it possible to store the object while maintaining the sterilized state.

According to the fourth embodiment described above, packing operation can be easily done in the sterilization apparatus as in the third embodiment, thus reducing the workload on the user.

### (Fifth Embodiment)

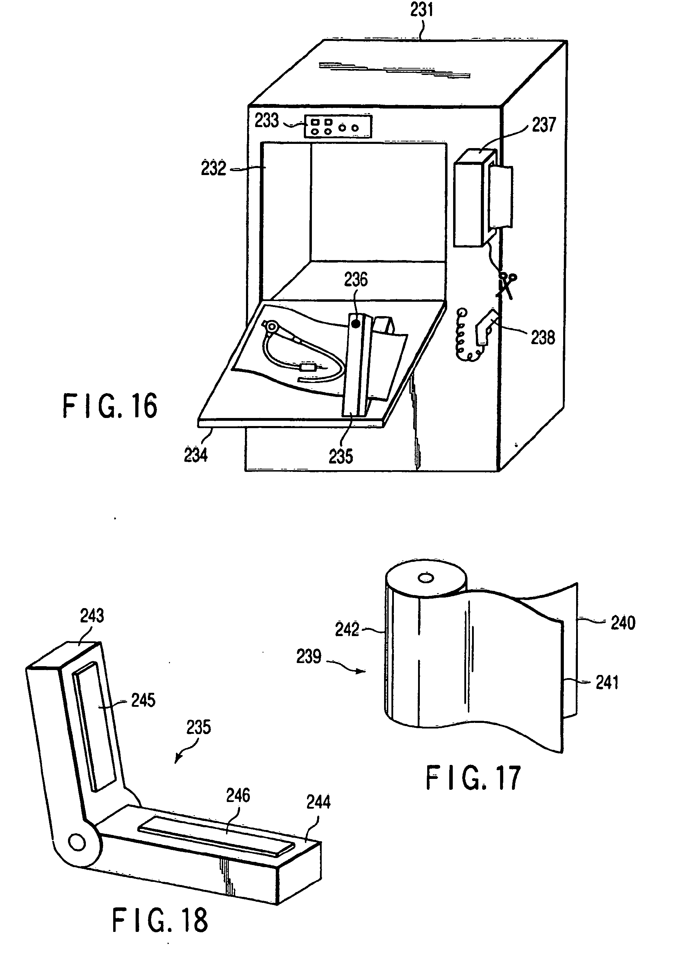

FIG. 16 is a view showing the arrangement of a sterilization apparatus according to the fifth embodiment of the present invention. Referring to FIG. 16, a sterilization apparatus 231 includes a chamber 232 and operation panel 233. The sterilization apparatus 231 is operated by operating the operation panel 233. The sterilization apparatus 231 includes a door 234, which makes it possible to open/close the chamber 232. As shown in FIG. 16, the door 234 opens to a horizontal position. When the door 234 is opened, a welding heat transfer portion 235 which seals a packing member which packs an object to be sterilized and is placed inside the apparatus appears.

The welding device 235 includes a switch 236. When the switch 236 is pressed, the welding heat transfer portion of the welding device 235 generates heat for a predetermined period of time. Referring to FIG. 16, reference numeral 237 denotes a roll packing member storing case. A packing member is cut in an arbitrary size to be used in accordance with the shape of an object to be sterilized. This apparatus also includes an air gun 238. This makes it possible to dry a cleaned object to be sterilized by blowing off water droplets adhering to the object before a sterilization process.

The air gun 238 communicates with a compressor (not shown) incorporated in the sterilization apparatus 231. When the air gun 238 is to be used, the compressor is operated to spray air from the air gun 238.

FIG. 17 is a view showing a packing member 239 in the fifth embodiment. The packing member 239 is comprised of a paper portion 240 and transparent film portion 241. The two portions are overlaid and supplied in the form of a roll. This packing member is cut in a necessary size to be used. Two sides 242 of the roll in the longitudinal direction are bonded to each other in advance and cut in a cylindrical shape.

FIG. 18 is an enlarged view of the welding device 235 (FIG. 16) described above. The welding device 235 is in the form of a hinge; an upper portion 243 vertically opens/closes, and a lower portion 244 is fixed to the door 234. The upper portion 243 includes a heat transfer portion 245. When the switch 236 of the welding device 235 is pressed, the heat transfer portion 245 generates heat for a predetermined period of time.

The lower portion 244 includes a packing 246 at a portion to which the heat transfer portion 245 is fitted when the upper portion 243 is closed. When the lower portion 244 comes into tight contact with the heat transfer portion 245, the packing member 239 is firmly clamped between them to weld and seal the packing member 239.

In this embodiment, when the object 215 is to be sterilized, the door 234 of the sterilization apparatus 231 is opened. Since the door 234 opens to a horizontal position, it serves as a workbench when the object 215 is to be packed. The cleaned object 215 is placed on the door 234, and when water droplets and the like adhere to the object, the object is dried by blowing air against it using the air gun 238.

The packing member 239 is then pulled from the roll packing member storing case 237 and cut in accordance with the size of the object 215. The object 215 is then stored into the packing member 239 through its opening portion. The opening portion of the packing member 239 is placed at a position where it is clamped between the upper portion 243 and lower portion 244 of the welding device 235. When the upper portion 243 is closed and the switch 236 is pressed, the heat transfer portion 245 of the welding device 235 generates heat to seal the packing member 239.

This operation is performed at two positions on the opening portion of the packing member 239 to pack the object 215 in the packing member 239. The packed object 215 is then placed in the chamber 232 of the sterilization apparatus 231. Thereafter, a sterilization process is performed by closing the door 234 and operating the operation panel 233.

According to the fifth embodiment described above, since the door of the sterilization apparatus can be used as a workbench near the sterilization apparatus, a work space can be efficiently used. In addition, an object to be sterilized can be packed in accordance with its size, and a plurality of packed objects to be sterilized can be processed depending on the capacity of the chamber.

According to the third to fifth embodiments described above, since an object to be sterilized can be packed near the sterilization apparatus and easily set in the apparatus, the working efficiency of sterilization can be improved.

### (Sixth Embodiment)

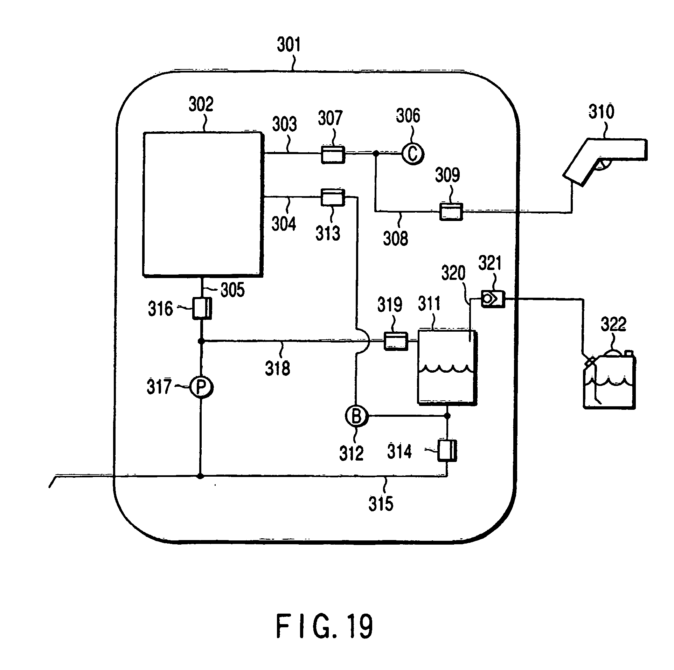

FIG. 19 is a view showing the arrangement of a sterilization apparatus 301 according to the sixth embodiment of the present invention. Referring to FIG. 19, reference numeral 302 denotes a chamber in which an object to be sterilized is stored and in which steam is supplied to perform a sterilization process. One end of each of an air supply piping 303, steam supply piping 304, and exhaust piping 305 is connected to the chamber 302. A compressor 306 is connected to the other end of the air supply piping 303. An air valve 307 is interposed midway along the air supply piping 303. One end of an air gun piping 308 is connected between the air valve 307 and the compressor 306.

The other end of the air gun piping 308 is connected to an air gun 310. Air is sprayed from the air gun 310 by operating an operation panel (not shown) to be used to, for example, blow off water droplets adhering to a cleaned object to be sterilized before a sterilization process.

The other end of the steam supply piping 304 is connected to the bottom portion of a water tank 311. A boiler 312 and steam valve 313 are interposed midway along the steam supply piping 304. In addition, one end of a water tank drain piping 315 is connected between the boiler 312 of the steam supply piping 304 and the water tank 311 via a water tank drain valve 314.

The other end of the water tank drain piping 315 is connected to the exhaust piping 305. The other end of the exhaust piping 305 whose one end is connected to the chamber 302 described above communicates with the outside of the sterilization apparatus 301 to discharge steam and drain from the chamber 302 to the outside. An exhaust valve 316 and vacuum pump 317 are interposed midway along the exhaust piping 305. One end of a suction piping 318 is connected between the exhaust valve 316 and the vacuum pump 317. The other end of the suction piping 318 is connected to an upper portion of the water tank 311 via a suction valve 319.

One end of a water supply piping 320 is open to the inside of the water tank 311. The other end of the water supply piping 320 communicates with the outside of the sterilization apparatus 301 via a check valve 321. The water supply piping 320 is inserted into the spout of a purified water tank 322, in which purified water to be supplied to the water tank 311 is stored, to execute a water supply step for the sterilization apparatus 301, thereby sucking purified water from the purified water tank 322 into the water tank 311.

The operation of the sterilization apparatus 301 described above will be described next. First of all, in order to perform a sterilization process, purified water required to generate steam used for sterilization is supplied into the water tank 311. The water supply piping 320 is inserted into the purified water tank 322 in which purified water is stored, and a water supply step is executed by operating the operation panel (not shown) of the sterilization apparatus 301. When the water supply step starts, the suction valve 319 opens, and the vacuum pump 317 operates to evacuate the water tank 311 via the suction piping 318.

With this operation, a negative pressure is created in the water tank 311 to suck purified water from.the purified water tank 322 via the water supply piping 320, thereby supplying the purified water into the water tank 311. The stored purified water is in an amount that allows a sterilization process to be performed a plurality of number of times. If the amount of water decreases in accordance with the frequency of use, a purified water supply step is performed. If purified water is aged without being used for a long period of time, the old purified water is discarded from the water tank 311 by performing a drain step, and new purified water is supplied to perform a sterilization process.

When a drain step is started by operating the operation panel (not shown), the water tank drain valve 314 opens to discard old purified water from the water tank 311 to the outside of the sterilization apparatus 301 via the water tank drain piping 315 and exhaust piping 305.

After purified water is stored in the water tank 311, an object to be sterilized is placed in the chamber 302, and a sterilization step is started by operating the operation panel (not shown). When the step starts, the steam valve 313 opens, and purified water supplied from the water tank 311 is heated by the boiler 312 to produce steam. The produced steam is supplied to the chamber 302 via the steam supply piping 304.

The interior of the chamber 302 is monitored by a pressure sensor and temperature sensor (not shown). When the temperature in the chamber 302 reaches a predetermined sterilization temperature, the steam valve 313 closes, and the boiler 312 stops, thus stopping the supply of steam. The interior of the chamber 302 is held at the predetermined sterilization temperature for a predetermined period of time to sterilize the object.

When the predetermined period of time elapses and sterilization is complete, the exhaust valve 316 opens and the vacuum pump 317 operates to exhaust steam from the chamber 302. The vacuum pump 317 evacuates the chamber 302 to a negative pressure to exhaust steam in the chamber 302, and promotes drying of moisture away from the object. When a predetermined period of time elapses afterward, the vacuum pump 317 stops and the air valve 307 opens. In addition, the compressor 306 operates to supply air into the chamber 302 via the air supply piping 303. As a consequence, the pressure in the chamber 302 returns to atmospheric pressure.

This operation promotes drying of the object and cools it. When a predetermined period of time has elapsed, the operation panel 6 stops, and a series of operations in the sterilization step is terminated.

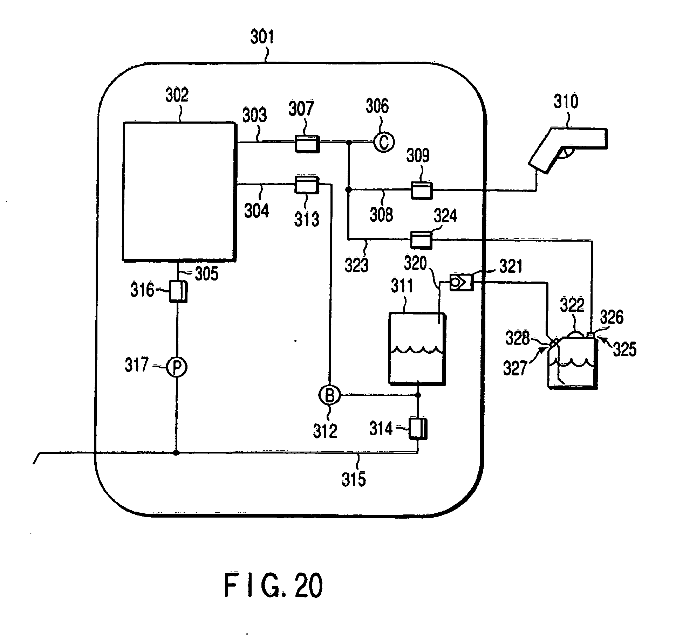

A method of supplying water to the water tank of the sterilization apparatus may be executed by the arrangement shown in FIG. 20. More specifically, as compared with the arrangement shown in FIG. 19, instead of not having the suction piping, one end of an air supply piping 323 is connected between the compressor 306 and the air valve 307, and the other end is connected, via a way air supply valve 324, to a ventilation hole 325 of the purified water tank 322 in which purified water to be supplied to the water tank 311 is stored. The air supply piping 323 is connected to the ventilation hole 325 via a connector 326 to supply air into the purified water tank 322 without any leakage.

In addition, a connector 328 is inserted midway along the water supply piping 320 inserted in a spout 327 of the purified water tank 322 to keep the airtightness of the purified water tank 322 connected to the spout 327. In this arrangement, when purified water is to be supplied to the water tank 311, a water supply step is started by operating an operation panel (not shown). When the step is started, the way air supply valve 324 opens and the compressor 306 operates. As a consequence, air is supplied to the purified water tank 322 via the air supply piping 323, and purified water in the purified water tank 322 is supplied by this pressure to the water tank 311 via the water supply piping 320.

### Industrial Applicability

According to the present invention, a work space can be effectively used in the field of medical practice, and operation before sterilization is facilitated. In addition, sanitary supervision can be enhanced.

In addition, the preparation time for the storage of an endoscope into the autoclave apparatus can be shortened, and the ease of use for a user improves. In addition, efficient endoscopic examination can be executed.

Furthermore, since packing work for an object to be sterilized is performed near the sterilization apparatus, the working efficiency of sterilization can be improved.

## Claims

1. A sterilization apparatus (50, 101, 201, 231) comprising:
a chamber (2, 102, 202, 232) in which an object (4, 113, 215) to be sterilized is stored and a sterilization process is performed; and
a work-space forming unit connected to the apparatus and adapted to form a work-space for execution by an operator of a process of storing said object in the chamber upon setting the object in a predetermined storage state ;
**characterized in that** the work-space forming unit comprises :
a workbench (3, 114, 204, 234) which may be stored/locked in the chamber and may be pulled out therefrom to be set at a predetermined position in a pulled-out state, wherein the workbench allows the object to be placed and allows execution of a process of storing the object in the chamber, and
a setting unit (12, 115, 205) adapted to hold the object in said predetermined state during execution of the sterilization process.

2. A sterilization apparatus according to claim 1, further comprising an operation member (5, 203, 223) which reduces a working load of returning the workbench from a pulied-out position to a storing position.

3. A sterilization apparatus according to claim 2, wherein the operation member comprises a foot-operated pedal.

4. A sterilization apparatus according to claim 1, wherein the setting unit includes a rotating surface (13) substantially parallel to a base surface of the workbench.

5. A sterilization apparatus according to claim 1, wherein the work-space forming unit includes a sterilization tray (112) which can be moved into and out of the chamber by a slide member (111), and the sterilization tray includes a sterilization tray lid (114) which forms said workbench.

6. A sterilization apparatus according to claim 1, wherein the setting unit (205) further comprises:
packing means for packing the object, and
sealing means for sealing the packing means.

7. A sterilization apparatus according to claim 6, wherein the packing means includes a seat portion (206) upon which the object and a packing member (216) which packs the object are placed, and wherein the sealing means comprises a lid portion (207) which seals and locks the packing member (216) packing the object, and a hinge portion 208 which allows the lid portion to be opened/closed.

8. A sterilization apparatus according to claim 7, wherein the lid portion (207) further includes a welding heat transfer portion (209) for sealing the packing member (216), and a hook portion (210) for fixing the welding heat transfer portion on a seal portion when the lid portion (207) is closed.

9. A sterilization apparatus according to claim 1, which includes a door for opening/closing the chamber, and in which a welding heat transfer portion for sealing a packing member which packs the object is placed inside the door, wherein a packing is mounted on a portion in the chamber to which the welding heat transfer portion is fitted, and the packing member is welded/sealed by bringing the packing into tight contact with the welding heat transfer portion with the packing member being clamped therebetween when the door is closed.

10. A sterilization apparatus according to claim 1, which includes a door for opening/closing the chamber, and in which a welding device for sealing a packing member which packs the object is placed inside the door, wherein the welding device (235) has a hinge shape including an upper portion (243) which opens/closes in a vertical direction and a lower portion (244) fixed to the door, and wherein a heat transfer portion (245, 246) is provided for the upper portion, the packing (246) is formed on the lower portion which is fitted to the upper portion when the upper portion closes, and the packing member is welded/sealed by clamping the packing member between the heat transfer portion and the packing.

## Patentansprüche

1. Sterilisierungsvorrichtung (50, 101, 201, 301), mit:
einer Kammer (2, 102, 202, 232), in der ein zu sterilisierendes Objekt (4, 113, 215) eingebracht ist und ein Sterilisierungsprozess ausgeführt wird; und
einer einen Arbeitsraum bildenden Einheit, die mit der Vorrichtung verbunden und so ausgeführt ist, dass sie einen Arbeitsraum bildet, in dem ein Bediener einen Prozess der Einbringung des Objektes in die Kammer ausführt, nachdem er das Objekt in einen vorgegebenen Zustand zur Einbringung versetzt hat;
**dadurch gekennzeichnet, dass** die den Arbeitsraum bildende Einheit aufweist:
eine Arbeitsplattform (3, 114, 204, 234), die in der Kammer untergebracht/arretiert und aus dieser herausgezogen werden kann, um in einer vorgegebenen Position im herausgezogenen Zustand festgestellt zu werden, in dem das Objekt auf der Arbeitplattform platziert und der Prozess des Einbringen des Objekts in die Kammer ausgeführt werden kann; und
eine Feststelleinheit (12, 155, 205), die so ausgeführt ist, dass sie das Objekt während der Ausführung des Sterilisationsprozesses im vorgegebenen Zustand hält.

2. Sterilisierungsvorrichtung nach Anspruch 1, ferner ein Betätigungselement (5, 203, 223) aufweisend, das die Betriebslast beim Zurückbewegen der Arbeitsplattform von der herausgezogenen Position in eine eingebrachte Position verringert.

3. Sterilisierungsvorrichtung nach Anspruch 2, bei der das Betätigungselement ein fußbetätigtes Pedal aufweist.

4. Sterilisierungsvorrichtung nach Anspruch 1, bei der die Feststelleinheit eine rotierende Oberfläche (13) enthält, die im Wesentlichen parallel zur Basisoberfläche der Arbeitsplattform liegt.

5. Sterilisierungsvorrichtung nach Anspruch 1, bei der die den Arbeitsraum bildende Einheit eine Sterilisierungswanne (112) enthält, die durch ein Gleitelement (111) in die und aus der Kammer bewegt werden kann, und die Sterilisierungswanne einen Sterilisierungswannendeckel (114) enthält, der die Arbeitsplattform bildet.

6. Sterilisierungsvorrichtung nach Anspruch 1, bei der die Feststelleinheit (205) ferner aufweist:
ein Verpackungsmittel zum Verpacken des Objekts; und
ein Dichtmittel zum Versiegeln des Verpackungsmittels.

7. Sterilisierungsvorrichtung nach Anspruch 6, bei der das Verpackungsmittel einen Sitzabschnitt (206) enthält, auf dem das Objekt und ein Verpackungselement (216), das das Objekt verpackt, platziert sind, und bei der das Dichtmittel einen Deckelabschnitt (207) aufweist, der das das Objekt verpackende Verpackungselement (216) versiegelt und verschließt, sowie einen Gelenkabschnitt (208), der das Öffnen/Schließen des Deckelabschnitts gestattet.

8. Sterilisierungsvorrichtung nach Anspruch 7, bei der der Deckelabschnitt (207) ferner einen Schweißwärme-Übertragungsabschnitt (209) zum Versiegeln des Verpackungselements (216) und einen Hakenabschnitt (210) zum Befestigen des Schweißwärme-Übertragungsabschnitts auf einem Versiegelungsabschnitt bei geschlossenem Deckelabschnitt (207) enthält.

9. Sterilisierungsvorrichtung nach Anspruch 1, die eine Tür zum Öffnen/Schließen der Kammer enthält, und bei der der Schweißwärme-Übertragungsabschnitt zum Versiegeln des Verpackungselements, das das Objekt verpackt, im Innern der Tür angeordnet ist, wobei die Verpackung an einem Abschnitt der Kammer angebracht ist, auf dem der Schweißwärme-Übertragungsabschnitt sitzt, und das Verpackungselement verschweißt/versiegelt wird, indem die Verpackung in innigen Kontakt mit dem Schweißwärme-Übertragungsabschnitt gebracht wird, wenn das Verpackungselement bei geschlossener Tür dazwischen eingeklemmt ist.

10. Sterilisierungsvorrichtung nach Anspruch 1, die eine Tür zum Öffnen/Schließen der Kammer enthält, und bei der ein Schweißgerät zum Versiegeln eines Verpackungselements, das das Objekt verpackt, im Innern der Tür angeordnet ist, wobei das Schweißgerät (235) eine Gelenkform mit einem oberen Abschnitt (243), der sich in senkrechter Richtung öffnet/ schließt, und einem an der Tür befestigten unteren Abschnitt (244) hat und wobei ein Wärmeübertragungsabschnitt (245) für den oberen Abschnitt vorgesehen ist, die Verpackung (246) auf dem unteren Abschnitt, der am oberen Abschnitt angebracht ist, gebildet wird, wenn sich der obere Abschnitt schließt, und das Verpackungselement verschweißt/versiegelt wird, indem das Verpackungselement zwischen dem Wärmeübertragungsabschnitt und der Verpackung eingeklemmt wird.

## Revendications

1. Appareil de stérilisation (50, 101, 201, 231) comprenant :
une enceinte (2, 102, 202, 232) dans laquelle un objet (4, 113, 215) à stériliser est stocké et un procédé de stérilisation est effectué ; et
une unité formant un espace de travail connectée à l'appareil et destiné à former un espace de travail pour l'exécution par un opérateur d'un procédé de stockage dudit objet dans l'enceinte lors de la mise en place de l'objet dans un état de stockage prédéterminé ;
**caractérisé en ce que** l'unité formant espace de travail comprend :
un plan de travail (3, 114, 204, 234) qui peut être stocké/verrouillé dans l'enceinte et retiré de celle-ci pour être placé à une position prédéterminée dans un état retiré, dans lequel le plan de travail permet que l'objet soit placé et permet l'exécution d'un processus de stockage de l'objet dans l'enceinte, et
une unité de mise en place (12, 115, 205) destiné à maintenir l'objet dans ledit état prédéterminé pendant l'exécution du procédé de stérilisation.

2. Appareil de stérilisation selon la revendication 1, comprenant en outre un élément opérationnel (5, 203, 223) qui réduit une charge de travail de retour du plan de travail d'une position retirée à une position de stockage.

3. Appareil de stérilisation selon la revendication 2, dans lequel l'élément opérationnel comprend une pédale commandée au pied.

4. Appareil de stérilisation selon la revendication 1, dans lequel l'unité de mise en place comprend une surface rotative (13) sensiblement parallèle à une surface de base du plan de travail.

5. Appareil de stérilisation selon la revendication 1, dans lequel l'unité formant un espace de travail comprend un plateau de stérilisation (112) qui peut être entré et sorti de l'enceinte par un élément coulissant (111), et le plateau de stérilisation comprend un couvercle du plateau de stérilisation (114) qui constitue ledit plan de travail.

6. Appareil de stérilisation selon la revendication 1, dans lequel l'unité de mise en place (205) comprend en outre :
des moyens d'emballage pour emballer ledit objet, et
des moyens de fermeture étanche pour fermer de façon étanche le moyen d'emballage.

7. Appareil de stérilisation selon la revendication 6, dans lequel le moyen d'emballage comprend une partie de siège (206) sur laquelle l'objet et un élément d'emballage (216) qui emballe l'objet sont placés, et dans lequel le moyen de fermeture étanche comprend une partie de couvercle (207) qui ferme de façon étanche et verrouille l'élément d'emballage (216) emballant l'objet, et une partie charnière (208) qui permet à la partie de couvercle d'être ouverte/fermée.

8. Appareil de stérilisation selon la revendication 7, dans lequel la partie de couvercle (207) comprend en outre une partie de transfert thermique de soudage (209) pour fermer de façon étanche l'élément d'emballage (216), et une partie crochet (210) pour fixer la partie de soudage par transfert thermique sur une partie de fermeture étanche lorsque la partie de couvercle (207) est fermée.

9. Appareil de stérilisation selon la revendication 1, qui comprend une porte pour ouvrir/fermer l'enceinte, et dans lequel une partie de soudage par transfert thermique pour fermer de façon étanche un élément d'emballage qui emballe l'objet est placée dans la porte, dans lequel un emballage est monté sur une partie dans l'enceinte sur laquelle la partie de soudage par transfert thermique est disposée, et l'élément d'emballage est soudé/fermé de façon étanche en mettant l'emballage en contact étroit avec la partie de soudage par transfert thermique avec l'élément d'emballage étant serré entre ceux-ci lorsque la porte est fermée.

10. Appareil de stérilisation selon la revendication 1, qui comprend une porte pour ouvrir/fermer l'enceinte, et dans lequel un dispositif de soudage pour fermer de façon étanche un élément d'emballage qui emballe l'objet est placé dans la porte, dans lequel le dispositif de soudage (235) a une forme de charnière comprenant une partie supérieure (243) qui s'ouvre/se ferme dans une direction verticale et une partie inférieure (244) fixée sur la porte, et dans lequel une partie de transfert thermique (245, 246) est prévue pour la partie supérieure, l'emballage (246) est formé sur la partie inférieure qui est disposée sur la partie supérieure lorsque la partie supérieure se ferme, et l'élément d'emballage est soudé/fermé de façon étanche en serrant l'élément d'emballage entre la partie de transfert thermique et l'emballage.
